(19) Europäisches Patentamt
European Patent Office
Office européen des brevets

(11) Publication number: 0 162 592 B1

(12) # EUROPEAN PATENT SPECIFICATION

(45) Date of publication of patent specification: 22.03.89

(21) Application number: 85302853.8

(22) Date of filing: 24.04.85

(51) Int. Cl.$^4$: C 07 D 491/052, A 61 K 31/40 // (C07D491/052, 311:00, 209:00)

(54) Heterocyclic amino compounds.

(30) Priority: 24.04.84 GB 8410459

(43) Date of publication of application: 27.11.85 Bulletin 85/48

(45) Publication of the grant of the patent: 22.03.89 Bulletin 89/12

(84) Designated Contracting States: BE CH DE FR GB IT LI NL

(56) References cited:
DE-A-2 356 900
FR-A-2 534 921

PATENT ABSTRACTS OF JAPAN, vol. 8, no. 134, 21st June 1984, (C-230) (1571);& JP-A-59-44389 (MITSUBISHI YUKA K.K.) 12-03-1984

(73) Proprietor: GLAXO GROUP LIMITED
Clarges House 6-12 Clarges Street
London W1Y 8DH (GB)

(72) Inventor: Cherry, Peter Clive
12 Thistledene Avenue
South Harrow, Middlesex (GB)

Inventor: Borthwick, Alan David
15 Temple Grove
London NW 11 (GB)

Inventor: Coles, Richard John
28 Norfolk Road
Uxbridge, Middlesex (GB)

Inventor: Burn, Derek
77 Sycamore Road Chalfont St. Giles
Buckinghamshire (GB)

(74) Representative: Kyle, Diana et al
ELKINGTON AND FIFE Beacon House 113 Kingsway
London WC2B 6PP (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to heterocyclic amino compounds. More specifically this invention relates to novel benzopyranylpyrrole derivatives, to processes for the preparation thereof, to pharmaceutical preparations containing them, and to their use in medicine.

The alpha (α)-adrenoreceptors of the sympathetic nervous system are classified pharmacologically into two sub-groups, namely $\alpha_1$ and $\alpha_2$. The $\alpha_2$- type are situated predominantly on the presynaptic terminals of noradrenergic neurones and are activated by the released neurotransmitter. Such activation results in a diminished release of noradrenaline on subsequent stimulation of the neurones, the $\alpha_2$-adrenoreceptors thus forming part of an autoinhibitory feedback mechanism for regulating the synaptic concentration of the neurotransmitter. A selective $\alpha_2$-adrenoreceptor antagonist would be expected to produce an increase in the synaptic concentrations of noradrenaline by blocking the autoinhibitory feedback mechanism and would thus be of potential value in human medicine for the treatment of disorders such as depression which are associated with a deficiency of noradrenaline at postsynaptic adrenoreceptors.

$\alpha_2$-Adrenoreceptors also occur at non-neuronal sites such as on blood-platelets, in pancreatic islet cells, on adipocytes and in the promixal tubules of the kidney. Activation of $\alpha_2$-adrenoreceptors at these sites leads to platelet aggregation, inhibition of insulin release, inhibition of lipolysis and retention of sodium respectively.

A selective $\alpha_2$-adrenoreceptor antagonist thus has a potential therapeutic use as an antidepressant either alone or in a complimentary combination with an established antidepressant, and in either treating or preventing conditions such as migraine, thrombosis, diabetes, obesity, hypertension, constipation, paralytic ileus and senile dementia.

We have now found that the compounds of formula (I) below and their physiologically acceptable salts have a selective $\alpha_2$-adrenoreceptor antagonist action.

The invention thus provides compounds of general formula (I)

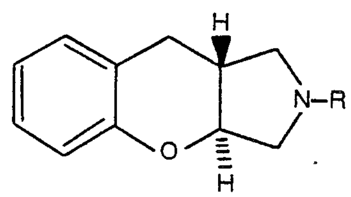

(I)

wherein R is a hydrogen atom or a group selected from $C_{1-6}$ alkyl (optionally substituted by $C_{3-7}$ cycloalkyl), $C_{3-6}$ alkenyl, $C_{3-6}$ alkynyl, $C_{3-7}$ cycloalkyl, aralkyl (in which the alkyl moiety contains 1—5 carbon atoms), and —CHO; and the physiologically acceptable salts thereof.

In general formula (I), the alkyl, alkenyl and alkynyl groups represented by R may be straight or branched chain groups.

When R contains a —C=C— or —C≡C— linkage this is not directly attached to the nitrogen atom. When R is alkyl it may be, for example, methyl, ethyl or propyl, methyl being preferred. When R is an alkyl group substituted by a $C_{3-7}$ cycloalkyl group it may be, for example, cyclopropyl $C_{1-3}$ alkyl such as cyclopropylmethyl. When R is alkenyl it may be, for example, allyl and when R is alkynyl it may be, for example, propynyl. When R is cycloalkyl it may be, for example, cyclopropyl. When R is an aralkyl group it may be, for example, phen$C_{1-5}$alkyl, such as benzyl.

Suitable physiologically acceptable salts are the acid addition salts formed with inorganic acids, for example hydrochlorides, hydrobromides, phosphates and sulphates, and with organic acids, for example citrates, tartrates, acetates, maleates and succinates. The hydrochlorides are particularly useful.

It will be appreciated that each compound of general formul (I) is a *trans* isomer and exists as two enantiomers. The structural formulae herein are to be understood to depict either enantiomer of each compound concerned as well as mixtures of the enantiomers, including racemates, even though the precise structure as set out only relates to one enantiomer.

A preferred group of compounds of general formula (I) is that wherein R is a hydrogen atom. Another preferred group of compounds of general formula (I) is that wherein R is a $C_{1-3}$ alkyl group, particularly a methyl or ethyl group.

Important compounds are (±)-*trans*-1,2,3,3a,9,9a-hexahydro[1]benzopyrano[2,3-c]pyrrole; its 3aS- and 3aR-isomers; and their physiologically acceptable salts, particularly the hydrochlorides.

The compounds of the invention have selective $\alpha_2$-adrenoreceptor antagonist action. The test for determining the $\alpha_2$-adrenoreceptor antagonist action is based on the ability to prevent the action of a selective $\alpha_2$-adrenoreceptor agonist such as clonidine or 5-bromo-N-(4,5-dihydro-1H-imidazol-2-yl)-6-quinoxalinamine-[R-(R*R*)]-2,3-dihydroxybutanedioate (UK 14304—18) on the rat field stimulated vas deferens preparation.

Clonidine and UK 14304—18 inhibit the twitch response of the rat isolated vas deferens to low frequency motor nerve stimulation. This inhibition is a consequence of activation of presynaptic adrenoreceptors of the $\alpha_2$-type. Antagonism of the effect of clonidine or UK 14304—18 is quantified by

measuring the parallel shift to the right of the inhibitory $\alpha_2$-adrenoreceptor agonist $\log_{10}$ (concentration)/response curve in the presence of increasing concentrations of the antagonist. Potency and competitiveness of antagonism are determined by the method of Arunlakshana & Schild (Br.J.Pharmac. 1959, *14* 48—58).

The α-adrenoreceptor-type selectivity of the compounds of general formula (I) is similarly assessed by measuring the ability to produce a parallel shift to the right of the $\log_{10}$ (concentration)/response curve for the $\alpha_1$-adrenoreceptor agonist phenylephrine. The $\alpha_1$-adrenoreceptor-mediated responses of phenylephrine measured were contractions of the rat isolated anococcygeus muscle (Leighton, Butz & Parameter, Eur.J.Pharmac., 1979, *58* 27—38).

The compounds of the invention are thus of interest in the treatment or prevention of migraine, thrombosis, diabetes, obesity, hypertension, constipation, paralytic ileus and senile dementia, and in particular for the treatment of depression.

The invention accordingly further provides compounds of general formula (I) and their physiologically acceptable salts for use in the therapy or prophylaxis of migraine, thrombosis, diabetes, obesity, hypertension, constipation, paralytic ileus and senile dementia and in particular depression. The compounds of the invention may be used either alone or with an additional active ingredient. Thus, for example, in the treatment of depression, the compounds of the invention may be used alone, or may be co-administered with an established antidepressant (e.g. desmethylimipramine, imipramine or amitriptyline) either in a single formulation or in separate formulations. The established antidepressant can be used in accordance with conventional practice.

The compounds according to the invention may be formulated in a conventional manner, optionally together with one or more other active ingredient, for administration by any convenient route for example for oral, rectal, intravenous or intramuscular administration. Oral administration is preferred.

Thus according to another aspect, the invention provides a pharmaceutical composition comprising a compound of general formula (1) and/or a physiologically acceptable salt thereof together with a physiologically acceptable carrier or excipient. The composition may optionally contain an additional active ingredient, for example an antidepressant such as desmethylimipramine, imipramine or amitriptyline.

For oral administration, the pharmaceutical composition may take the form of, for example, tablets, capsules, powders, solutions, syrups or suspensions prepared by conventional means with physiologically acceptable excipients.

Compositions for rectal administration may be in the form of suppositories using a conventional suppository excipient.

The compounds may be formulated for intravenous or intramuscular administration in dry form for reconstitution before use, or as a sterile solution or suspension.

A proposed daily dose for administration to man is 0.01 to 10 mg/kg, for example 0.05 to 3 mg/kg, which may be conveniently administered in 1 to 3 doses per day. The precise dose administered will of course depend on the age and condition of the patient. The daily dosage may conveniently be administered in the form of dosage units, each unit containing for example 0.01 to 3 mg/kg of active ingredient.

The compounds according to the invention may be prepared by a number of processes. In the following description the group R is as previously defined for general formula (I) except where otherwise indicated.

According to a first example (A), a compound of general formula (I) may be prepared by amination of a compound of general formula (II)

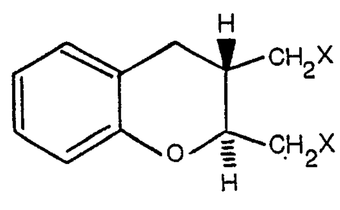

(II)

where X is a leaving group such as a halogen atom, (e.g. chlorine, bromine or iodine), or a hydrocarbylsulphonyloxy group e.g. methylsulphonyloxy, with ammonia, aqueous ammonia or an amine of formula $RNH_2$ where R is as previously defined except that R is not a hydrogen atom or the group —CHO.

In a particular embodiment of this process, following the amination reaction, the resulting compound of general formula (I) or a salt thereof, may be converted into another compound of general formula (I). Thus, for example, when R is arylmethyl, the amination reaction may optionally be followed by removal of the arylmethyl group to yield a compound of formula (I) where R is a hydrogen atom.

The amination reaction is conveniently effected at an elevated temperature e.g. under reflux or in a sealed tube at e.g. 110°C, preferably in the presence of a suitable base e.g. sodium hydride or an alkali metal hydroxide such as sodium hydroxide or in the presence of an excess of the amine $RNH_2$, optionally in the presence of a solvent such as an ether e.g. dioxan, chlorinated hydrocarbon e.g. chloroform or an alcohol e.g. ethanol. Optional removal of an arylmethyl group may be carried out for example by hydrogenolysis or, where appropriate, under acidic conditions, as described below.

According to another example (B), a compound of general formula (I) where R represents a hydrogen atom may be prepared by deprotection of a corresponding compound where R represents a protecting group. Suitable protecting groups include, for example, arylmethyl and acyl groups. Conventional deprotection procedures may be used. For example, where appropriate an arylmethyl group (e.g. benzyl) may be removed by hydrogenolysis using, for example, hydrogen in the presence of a catalyst, such as platinum or palladium on a support (e.g. charcoal), in a solvent such as an alcohol e.g. methanol. Alternatively, where appropriate, an arylmethyl group (e.g. trityl) may be removed under acidic conditions, using for example an acid such as trifluoroacetic acid, formic acid or hydrobromic acid. Acyl groups may be removed by hydrolysis using an acid such as mineral acid or a base such as an alkali metal hydroxide as appropriate. The protected starting materials for this process may be prepared using standard methods for the protection of amines, for example as described by J. F. W McOmie in 'Protective Groups in Organic Chemistry' (Plenum Press, 1973).

According to a further example (C), a compound of general formula (I) where R represents an alkyl group may be prepared by reduction of the corresponding compound in which R is an acyl group using a reducing agent such as lithium aluminium hydride or diborane in a suitable solvent such as ether or tetrahydrofuran at an elevated temperature e.g. reflux. Suitable acyl groups are, for example, formyl, acetyl, or carbonyloxyalkyl e.g. carbonyloxymethyl. The intermediate starting materials for this reaction may be prepared by acylation using conventional methods, for example by reaction of the compound of formula (I) in which R represents a hydrogen atom, with an acid chloride, acid anhydride, or ester.

The product of any of the processes (A), (B) and (C) described above may be subjected to one or two further reactions comprising:

(D)(i) converting the resulting compound of general formula (I) or a salt thereof into another compound of general formula (I); and/or

(D)(ii) converting a compound of general formula (I) or a salt thereof into a physiologically acceptable salt thereof.

Thus, it is also possible to prepare a compound of general formula (I) by a process comprising interconversion of another compound of general formula (I).

For example, a compound of general formula (I) in which R is a hydrogen atom may be converted by alkylation to a compound of general formula (I) in which R is an alkyl, substituted alkyl, alkenyl, alkynyl or aralkyl group. Conventional alkylation procedures may be used, for example reductive alkylation using an appropriate aldehyde with a complex metal hydride such as sodium or potassium borohydride or sodium cyanoborohydride in a suitable solvent such as an alcohol e.g. methanol.

Alternatively, the alkylation may be performed with an alkylating agent RX (where R is an alkyl, substituted alkyl, alkenyl, alkynyl or aralkyl group and X is a leaving group such as a halogen atom e.g. chlorine or bromine, or a hydrocarbylsulphonoxy group e.g. *p*-toluenesulphonyloxy) preferably in the presence of a base, such as potassium carbonate, optionally in a solvent such as an alcohol, e.g. ethanol.

Another example of this embodiment is the preparation of a compound of general formula (I) where R is a group —CHO, which may be prepared by formylation of a corresponding compound of formula (I) in which R is a hydrogen atom using an appropriate formylating agent such as a formyl ester, e.g. an alkyl formate such as methyl formate.

Physiologically acceptable salts of the compounds of general formula (I) may be prepared by reacting the free base of formula (I) or a salt thereof with an appropriate acid, such as hydrogen chloride in the presence of a suitable solvent e.g. ethyl acetate, ether or $CH_2Cl_2$ to obtain the desired physiologically acceptable salt.

The intermediate compounds of general formula (II) may be prepared by reaction of a corresponding diol of formula (III).

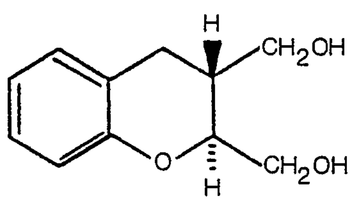

(III)

with a halide of formula $X_1A$ (where $X_1$ is a hydrocarbylsulphonyl group e.g. methylsulphonyl and A is a halogen atom e.g. a chlorine atom) in the presence of a base e.g. triethylamine in a solvent such as dichloromethane; or with a halogenating agent such as thionyl chloride, phosphorous tribromide or hydrogen iodide.

The diol of formula (III) may be prepared by the following sequence of reactions:

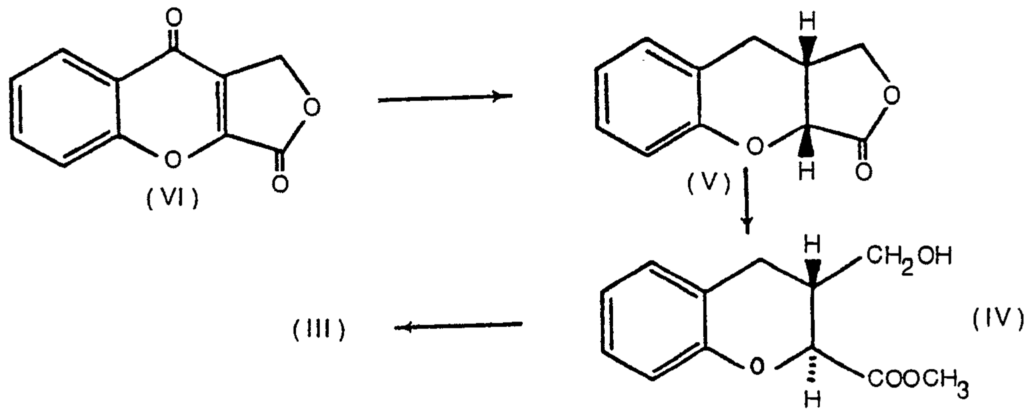

(VI) (V) (IV) (III)

Reduction of the dione (VI — Puetzer *et al.*, J.A.C.S. 1945, *67*, 832) using hydrogen and palladium on charcoal in glacial acetic acid yields the lactone (V), which is converted to the ester (IV) by reaction with potassium carbonate and methanol. Reduction of the ester (IV) using lithium aluminium hydride in tetrahydrofuran then gives the required diol (III).

A specific enantiomer of general formula (I) may be prepared by resolution of a mixture of enantiomers of formula (I) by conventional methods, e.g. by salt formation with an optically active acid followed by separation of the resulting diastereoisomeric salts, e.g. by fractional crystallisation. Alternatively, resolution may be effected at any suitable intermediate stage.

The following examples illustrate the invention. All temperatures are in °C.

Intermediate 1

(±)-cis-9,9a-Dihydro-1H-furo[3,4-b][1]benzopyran-3-(3aH)-one

A suspension of 1H-furo[3,4-b][1]benzopyran-3,9-dione (6.1 g) in glacial acetic acid (300 ml) was hydrogenated over 10% palladium on charcoal (1 g) until hydrogen uptake ceased. The catalyst was filtered off and the solvent evaporated from the filtrate to give an oil. The oil was dissolved in ethyl acetate and the solution washed with aqueous sodium bicarbonate and brine. The organic layer was separated, dried ($MgSO_4$) and the solvent evaporated. Crystallisation of the residue from ethyl acetate/ether gave the *title compound* (3.85 g) m.p. 107—111°.

Intermediate 2

(±)-trans-3,4-Dihydro-3-(hydroxymethyl)-2H-1-benzopyran-2-carboxylic acid, methyl ester

To Intermediate 1 (0.19 g) in dry methanol (2 ml) was added anhydrous potassium carbonate (0.41 g), the mixture was stirred at room temperature for two days, then partitioned between ethyl acetate and brine. The organic layer was separated, dried ($MgSO_4$) and the solvent evaporated to give a solid. Recrystallisation from ether/petroleum ether b.p. 60—80° gave the *title compound* (0.04 g) m.p. 84—85°.

Intermediate 3

(±) trans-3,4-Dihydro-2H-1-benzopyran-2,3-dimethanol

A solution of Intermediate 2 (1.4 g) in dry tetrahydrofuran (30 ml) was added to a stirred, ice cooled, mixture of lithium aluminium hydride (0.24 g) and tetrahydrofuran (20 ml) over 0.3 hr. After 1.5 hr a further quantity of lithium aluminium hydride (0.12 g) was added and the mixture was stirred at room temperature. After an additional 1 hr the reaction was cooled to 0—5° and saturated ammonium chloride solution (10 ml) added dropwise. The mixture was filtered and the gummy solid washed well with tetrahydrofuran. The combined filtrate and washings were evaporated and the residue partitioned between ethyl acetate and brine. The aqueous phase was separated and twice extracted with ethyl acetate. The combined extracts were dried ($MgSO_4$) and the solvent evaporated. The residue was crystallised from ether/petroleum ether b.p. 60—80° to give the *title compound* (1.05 g) m.p. 82—85°.

Intermediate 4

(±)-trans-3,4-Dihydro-2H-1-benzopyran-2,3-dimethanol, bis (methanesulphonate)

To a stirred mixture of Intermediate 3 (1.02 g) and triethylamine (2.25 ml) in dry dichloromethane (25 ml) at 0—5° was added methanesulphonyl chloride (0.93 ml) in dichloromethane (25 ml) over 15 min. After 20 min the solution was washed successively with water, 2N-hydrochloric acid, saturated sodium bicarbonate solution, and water. The organic phase was separated, dried ($MgSO_4$), and the solvent evaporated. Trituration of the residue with ether gave the *title compound* (1.67 g) m.p. 96—98°.

Example 1

(±)-trans-1,2,3,3a,9,9a-Hexahydro-2-(phenylmethyl)[1]benzopyrano[2,3-c]pyrrole

Intermediate 4 (1.49 g) and benzylamine (2.32 ml) were mixed and heated to 120° under nitrogen for 45 min. Water (10 ml) was added to the cold mixture to give a white solid, which was filtered off, washed with cold water and dried *in vacuo* over $P_2O_5$ to give the *title compound* (1.11 g), m.p. 68—70°, NMR ($CDCl_3$) τ 2.6—2.85 (5H, m, phenyl), 2.85—3.3 (4H, multiplets, aromatic), 5.96 (1H, m, 3a—H), 6.15 and 6.24 (2H, ABq, J12, $CH_2$Ph), 6.8—7.5 (6H, multiplets, 1—$H_2$, 3—$H_2$, and 9—$H_2$), and 7.67 (1H, m, 9a—H).

Example 2

(±)-trans-1,2,3,3a,9,9a-Hexahydro[1]benzopyrano[2,3-c]pyrrole, hydrochloride

To the compound of Example 1 (1.09 g) in methanol (40 ml) was added 10.9 M hydrogen chloride in methanol (0.41 ml) and 10% palladium on charcoal (0.1 g). The mixture was hydrogenated at 50° until hydrogen uptake ceased. The catalyst was removed by filtration and the filtrate evaporated to give a white solid. Recrystallisation from propan-1-ol gave the *title compound* (0.56 g) m.p. darkens from 225° and melts 265—270° (d), NMR (DMSO—$d_6$) τ 2.7—3.2 (4H, multiplets, aromatic), 5.85 (1H, m, 3a—H), 6.2—6.5 and 6.8—7.3 (6H, multiplets, 1—$H_2$, 3—$H_2$ and 9—$H_2$), and 7.77 (1H, m, 9a—H).

Pharmaceutical Examples

In the following examples, 'Active Ingredient' refers to (±) *trans*-1,2,3,3a,9,9a-hexahydro[1]benzopyrano[2,3-c]pyrrole hydrochloride. Other compounds of the invention may be formulated in similar fashion.

| | | |
|---|---|---|
| 1. | Oral Capsule | per capsule |
| | Active Ingredient | 50 mg |
| | Magnesium stearate | 0.5 mg |
| | Anhydrous lactose | 50 mg |

Blend the active ingredient with the lactose and magnesium stearate. Fill the blend into appropriate size hard gelatin capsules (lock fitting type) on an automatic capsule filling machine).

| | | |
|---|---|---|
| 2. | Oral Syrup | per 5 ml dose |
| | Active Ingredient | 50 mg |
| | Sodium citrate | 25 mg |
| | Citric acid | to pH 4.5 |
| | Sunset yellow FCF (Dye) | 0.25 mg |
| | Methyl hydroxybenzoate sodium | 5.0 mg |
| | Propyl hydroxybenzoate sodium | 2.0 mg |
| | Liquid orange flavour | qS |
| | Sucrose | 3.25 g |
| | Purified water | to 5.0 ml |

Dissolve the sucrose in a minimum quantity of water. Add a concentrated solution of sodium citrate with stirring and adjust the pH to 4.5 with citric acid. With continued stirring, add a 10% aqueous solution of the active ingredient, followed by a solution of the dye, a solution of the hydroxybenzoates and lastly the flavour. Adjust almost to volume with water and stir. Check the pH and adjust to 4.5 with citric acid if necessary. Make up to volume with water.

3. Oral Tablet

| | per tablet |
|---|---|
| Active Ingredient | 50 mg |
| Polyvinylpyrrolidone | 4.0 mg |
| Sodium starch glycollate | 10.0 mg |
| Magnesium stearate | 2.0 mg |
| Lactose to tablet core weight to | 200 mg |

Blend the active ingredient with the lactose. Add a sufficient quantity of polyvinylpyrrolidone solution to produce a damp mass suitable for granulation. Prepare the granules and dry using a tray or fluid bed dryer. Pass through a sieve, blend with the remaining ingredients and compress into 8 mm diameter tablets on a tablet machine.

Film coat the tablet cores with hydroxypropyl methyl cellulose or similar film forming material, using either an aqueous or non-aqueous solvent system. A plasticizer and suitable colour may be included in the film coating solution.

Activity

The selective $\alpha_2$-adrenoreceptor antagonist action of the product of Example 2 was demonstrated using the following tests:

(1) $\alpha_2$-Adrenoreceptor antagonist action

Compounds were tested for their ability to prevent the action of the selective $\alpha_2$-adrenoreceptor agonist clonidine on the rat field stimulated vas deferens preparation. Antagonism of the effect of clonidine was quantified by measuring the parallel shift to the right of the inhibitory clonidine $\log_{10}$ (concentration)/response curve in the presence of increasing concentrations of the test compound.

(2) $\alpha_1$-Adrenoreceptor antagonist action

Compounds were tested for their ability to prevent the action of $\alpha_1$-adrenoreceptor agonist phenylephrine in the rat isolated anococcygens muscle (Leighton, Butz & Parmeter, Eur. J. Pharmac., 1979 *58* 27—38). Antagonism of the effect of phenylephrine was quantified by measuring the parallel shift to the right of the phenylephrine $\log_{10}$ (concentration)/response curve in the presence of increasing concentrations of the test compound.

In (1) and (2) potency and competiveness of antagonism were determined by the method of Arunlakshana & Schild (Br. J. Pharmac. 1959, *14* 48—58).

In these tests, the $K_B$ value of the product of Example 2 at $\alpha_2$-adrenoreceptors was less than 0.1 µM and at $\alpha_1$-adrenoreceptors was greater than 10 µM ($K_B$ is defined as the concentration of test compound required to shift the $\log_{10}$ (concentration)/response curve of an agonist to the right by a factor of 2).

Toxicity

The compounds of the invention are in general non-toxic at therapeutically useful doses. Thus, the compounds of the examples showed no adverse effects when tested in mice at concentrations up to 50 mg/kg orally.

**Claims**

1. A compound of general formula (I)

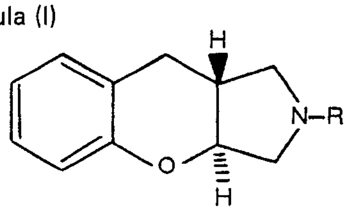

(I)

Wherein R is a hydrogen atom or a group selected from $C_{1-6}$ alkyl (optionally substituted by $C_{3-7}$ cycloalkyl), $C_{3-6}$ alkenyl, $C_{3-6}$ alkynyl, $C_{3-7}$ cycloalkyl, aralkyl (in which the alkyl moiety contains 1—5 carbon atoms) and CHO, provided that when R contains a —C=C— or —C≡C— linkage this is not directly attached to the nitrogen atom; and the physiologically acceptable salts thereof.

2. A compound according to claim 1, wherein, in the general formula (I), R is a $C_{1-3}$ alkyl group.

3. A compound according to claim 1, wherein in the general formula (I), R is a hydrogen atom.

4. A compound selected from (±)-*trans*-1,2,3,3a,9,9a-hexahydro[1]benzopyrano[2,3-c]pyrrole; its 3aS- and 3aR- isomers; and its physiologically acceptable salts.

5. A pharmaceutical composition comprising a compound of general formula (I) and/or a physiologically acceptable salt thereof according to any of claims 1 to 4 together with a physiologically acceptable carrier or excipient.

6. A pharmaceutical composition according to claim 5, which also comprises an established antidepressant.

7. A compound of general formula (I) or a physiologically acceptable salt thereof according to any of claims 1 to 5, for use in the therapy or prophylaxis of depression, migraine, thrombosis, diabetes, obesity, hypertension, constipation, paralytic ileus or senile dementia.

8. A process for the preparation of a compound of general formula (I) as defined in claim 1 or a physiologically acceptable salt thereof which comprises:—

(A) aminating a compound of general formula (II):

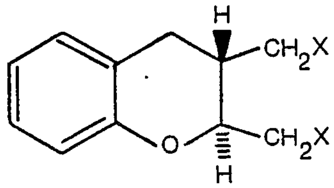

(II)

where X is a leaving group with ammonia, aqueous ammonia or an amine of formula $RNH_2$ where R is as defined for general formula (I) except that R is not a hydrogen atom or the group —CHO; or

(B) in order to prepare a compound of general formula (I) where R represents a hydrogen atom, deprotecting a corresponding compound where R represents a protecting group; or

(C) in order to prepare a compound of general formula (I) where R represents an alkyl group, reducing the corresponding compound where R represents an acyl group; and, if desired, subjecting the compound thus obtained to one or two further reactions comprising:

(D)(i) converting the resulting compound of general formula (I) or a salt thereof into another compound of general formula (I) and/or

(D)(ii) converting a compound of general formula (I) or a salt thereof into a physiologically acceptable salt thereof.

**Patentansprüche**

1. Verbindung der allgemeinen Formel (I)

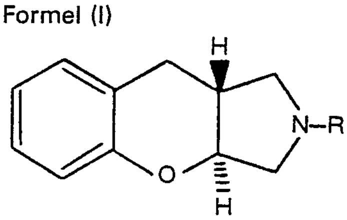

(I)

worin R ein Wasserstoffatom oder eine Gruppe, ausgewählt unter $C_{1-6}$-Alkyl (gegebenenfalls substituiert durch $C_{3-7}$-Cycloalkyl), $C_{3-6}$-Alkenyl, $C_{3-6}$-Alkinyl, $C_{3-7}$-Cycloalkyl, Aralkyl (worin der Alkyl-Molekülteil 1 bis 5 Kohlenstoffatome enthält) und CHO bedeutet, mit der Maßgabe, daß, wenn R eine —C=C— oder —C≡C— Bindung enthält, diese nicht direkt an das Stickstoffatom gebunden ist, und ihre physiologisch annehmbaren Salze.

2. Verbindung nach Anspruch 1, dadurch gekennzeichnet, daß in der allgemeinen Formel (I) R eine $C_{1-3}$-Alkylgruppe bedeutet.

3. Verbindung nach Anspruch 1, dadurch gekennzeichnet, daß in der allgemeinen Formel (I) R ein Wasserstoffatom bedeutet.

4. Verbindung, ausgewählt unter (±)-trans-1,2,3,3a,9,9a-Hexahydro[1]benzopyrano[2,3-c]pyrrol, ihren 3aS- und 3aR- Isomeren und ihren physiologisch annehmbaren Salzen.

5. Pharmazeutisches Präparat, dadurch gekennzeichnet, daß es eine Verbindung der allgemeinen Formel (I) und/oder ihr physiologisch annehmbares Salz nach einem der Ansprüche 1 bis 4 zusammen mit einem physiologisch annehmbaren Träger oder Verdünnungsmittel enthält.

6. Pharmazeutisches Präparat nach Anspruch 5, dadurch gekennzeichnet, daß es ebenfalls ein anerkanntes Antidepressivum enthält.

7. Verbindung der allgemeinen Formel (I) oder eines ihrer physiologisch annehmbaren Salze nach einem der Ansprüche 1 bis 5 für die Verwendung in der Therapie oder Prophylaxe von Depression, Migräne, Thrombose, Diabetes, Fettsucht, Hypertonie, Obstipation des paralytischen Darmverschlusses oder von seniler Dementia.

8. Verfahren zur Herstellung einer Verbindung der allgemeinen Formel (I) nach Anspruch 1 oder eines ihrer physiologisch annehmbaren Salze, dadurch gekennzeichnet, daß

(A) eine Verbindung der allgemeinen Formel (II):

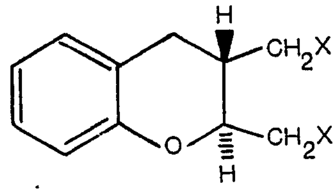

(II)

worin X eine abspaltbare Gruppe bedeutet, mit Ammoniak, wäßrigem Ammoniak oder einem Amin der Formel $RNH_2$, worin R die bei der allgemeinen Formel (I) gegebene Bedeutung besitzt, ausgenommen, daß R nicht Wasserstoff oder die Gruppe —CHO bedeutet, aminiert wird oder

(B) zur Herstellung einer Verbindung der allgemeinen Formel (I), worin R ein Wasserstoffatom bedeutet, von der entsprechenden Verbindung, worin R eine Schutzgruppe bedeutet, die Schutzgruppe abgespalten wird oder

(C) zur Herstellung einer Verbindung der allgemeinen Formel (I), worin R eine Alkylgruppe bedeutet, die entsprechende Verbindung, worin R eine Acylgruppe bedeutet, reduziert wird und gegebenenfalls die so erhaltene Verbindung einer oder zwei weiteren Reaktionen wie folgt unterworfen wird:

(D)(i) Umwandlung der entstehenden Verbindung der allgemeinen Formel (I) oder ihres Salzes in eine andere Verbindung der allgemeinen Formel (I) und/oder

(D)(ii) Umwandlung einer Verbindung der allgemeinen Formel (I) oder ihres Salzes in eines ihrer physiologisch annehmbaren Salze.

**Revendications**

1. Composé de formule générale (I)

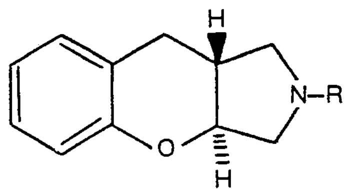

(I)

dans laquelle R est un atome d'hydrogène ou un groupe choisi parmi un alkyle $C_{1-6}$ (éventuellement substitué par un cycloalkyle $C_{3-7}$), un alkényle $C_{3-6}$, un alkynyle $C_{3-6}$, un cycloalkyle $C_{3-7}$, un aralkyle (dans lequel le motif alkyle contient de 1 à 5 atomes de carbone) et CHO, avec la condition que lorsque R contient une liaison —C=C— ou —C≡C— celle-ci n'est pas directement reliée à l'atome d'azote; et ses sels physiologiquement acceptables.

2. Composé selon la revendication 1, dans lequel, dans la formule générale (I), R est un groupe alkyle $C_{1-3}$.

3. Composé selon la revendication 1, dans lequel, dans la formule générale (I), R est un atome d'hydrogène.

4. Composé choisi parmi le (±)-*trans*-1,2,3,3a,9,9a-hexahydro[1]benzopyrano[2,3-c]pyrrole; ses isomères 3aS- et 3aR-; et ses sels physiologiquement acceptables.

5. Composition pharmaceutique comportant un composé de formule générale (I) et/ou un sel de celui-ci physiologiquement acceptable selon l'une quelconque des revendications 1 à 4, avec un support ou excipient physiologiquement acceptables.

6. Composition pharmaceutique selon la revendication 5, comportant également un antidépresseur reconnu.

7. Composé de formule générale (I) ou un sel physiologiquement acceptable de celui-ci selon l'une quelconque des revendications 1 à 5, pour emploi dans la thérapie ou la prophylaxie de la dépression, de la migraine, de la thrombose, du diabète, de l'obésité, de l'hypertension, de la constipation, de l'ileus paralytique ou de la démence sénile.

8. Procédé pour la préparation d'un composé de formule générale (I) tel que défini dans la revendication 1 ou un sel physiologiquement acceptable de celui-ci, qui comprend:

(A) l'amination d'un composé de formule générale (II):

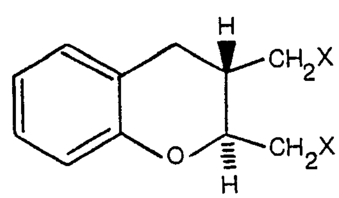

(II)

dans laquelle X est un groupe sortant avec de l'ammoniac, de l'ammoniaque ou une amine de formule $RNH_2$ où R est tel que défini pour la formule générale (I) sauf que R n'est pas un atome d'hydrogène ou le groupe —CHO; ou

(B) pour préparer un composé de formule générale (I) où R représente un atome d'hydrogène, la déprotection d'un composé correspondant où R représente un groupe protecteur; ou

(C) pour préparer un composé de formule générale (I) où R représente un groupe alkyle, la réduction du composé correspondant où R représente un groupe acyle; et, si on le désire, la soumission du composé ainsi obtenu à une ou deux réactions complémentaires comportant:

(D)(i) la transformation du composé résultant de formule générale (I) ou un sel de celui-ci en un autre composé de formule générale (I) et/ou

(D)(ii) la transformation d'un composé de formule générale (I) ou un sel de celui-ci en un sel de celui-ci physiologiquement acceptable.